# EUROPÄISCHE PATENTSCHRIFT

(11) **EP 3 142 724 B1**
(45) Veröffentlichungstag und Bekanntmachung des Hinweises auf die Patenterteilung: **21.08.2019**
(21) Anmeldenummer: 15723839.5
(22) Anmeldetag: 15.05.2015
(51) Int. Cl.: A61M 1/36, A61B 5/145, G01L 19/06

(54) **MEDIZINTECHNISCHE MESSVORRICHTUNG UND MESS-VERFAHREN**
MEDICAL TECHNOLOGY MEASURING DEVICE AND MEASUREMENT METHOD
DISPOSITIF DE MESURE MÉDICAL ET PROCÉDÉ DE MESURE

(30) Priorität: 15.05.2014 EP 14001728
(43) Veröffentlichungstag der Anmeldung: 22.03.2017
(60) Teilanmeldung: 19181061.3
(73) Patentinhaber: novalung GmbH, 74076 Heilbronn (DE)
(72) Erfinder: MAURER, Andreas, 72072 Tübingen (DE); FILIPON, Sven, 74080 Heilbronn (DE); BEURER, Matthias, 70469 Stuttgart (DE)
(74) Vertreter: Graf von Stosch, Andreas
(86) Internationale Anmeldenummer: PCT/EP2015/001001
(87) Internationale Veröffentlichungsnummer: WO 2015/172891

(56) Entgegenhaltungen:
- EP-A1- 0 688 531
- EP-A2- 0 685 721
- EP-A2- 0 762 084
- WO-A1-97/15228
- US-A1- 2007 261 496
- US-B1- 6 272 930

## Beschreibung

Die vorliegende Erfindung betrifft eine medizintechnische Messvorrichtung zur Messung einer Eigenschaft eines Fluids, insbesondere zur Druckmessung, umfassend eine Leitung, die sich entlang einer Mittenlängsachse erstreckt und eingerichtet ist, ein Fluid, insbesondere Blut, innerhalb einer von einer Wandung umgrenzten Längskavität zu führen; und eine Sensoreinrichtung mit einem Sensor, die eingerichtet ist, eine Eigenschaft des in der Längskavität geführten Fluids zu messen. Ferner betrifft die vorliegende Erfindung ein Verfahren zum Herstellen einer medizintechnischen Messvorrichtung, umfassend die Schritte: Bereitstellen einer Leitung mit einer Wandung; und Anordnen eines Sensors an der Leitung. Die vorliegende Erfindung betrifft insbesondere eine medizintechnische Messvorrichtung mit einzelnen Merkmalen des Anspruchs 1 sowie ein Herstellungsverfahren oder auch ein Messverfahren jeweils mit einzelnen Merkmalen des unabhängigen Verfahrensanspruchs.

Es sind Messvorrichtungen oder Sensoren bekannt, welche an einem Konnektor, Adapter oder an einem Zwischenstück zwischen einzelnen Abschnitten einer Leitung angeordnet sind. Die Leitung führt ein Fluid, insbesondere eine isotonische Kochsalzlösung oder andere kristalloide Infusionslösungen oder Blut, dessen Eigenschaft, insbesondere Druck erfasst werden soll. In solchen Messvorrichtungen besteht häufig das Risiko von Gerinnung (oder so genanntem Blut-Clotting) oder Hämolyse, insbesondere aufgrund von Kanten, Hinterschneidungen oder strömungstechnisch ungünstigen Übergängen. Die Verbindungselemente oder Konnektoren erhöhen auch das Risiko von Leckagen, insbesondere an Schnittstellen zwischen einem Konnektor und einem extrakorporalem Schlauchset oder im Messsystem, und müssen zusätzlich geprüft werden. Aus der Praxis ist insbesondere das Problem bekannt, dass z.B. an Luer-Anschlüssen Leckagen auftreten können. Speziell bei der extrakorporalen Zirkulation besteht dann das Risiko, dass in eine abführende Leitung, in welcher ein Unterdruck vorliegen kann, möglicherweise Luft eingesaugt und mit dem Fluid/Blut vermengt wird. Aus einer zuführenden Leitung, in welcher ein Überdruck vorliegen kann, wird möglicherweise an solchen Leckagen Fluid/Blut aus der Leitung herausgepresst. Die Sterilität der gesamten Vorrichtung kann dann nicht mehr sichergestellt werden. Auch muss der Konnektor oder Adapter an der Leitung montiert werden, und es muss eine möglichst sterile oder sterilisierbare Abdichtung, Verklebung oder sonstige Schnittstelle sichergestellt werden können.

Alternativ zu solchen in die Leitung eingekuppelten oder eingebundenen Messvorrichtungen oder Sensoren kann eine Messung, insbesondere Druckmessung, auch außerhalb der Leitung erfolgen. Der entsprechende Sensor kann einen Druck dann mittelbar erfassen, z.B. mittels einer Wassersäule. Bei dieser Art Druckmessung ist jedoch ein so genannter Priming-Vorgang erforderlich, um die Druckmessung starten zu können. Beim Priming erfolgt ein Füllen des Schlauchsystems mit einer kristalloiden Lösung wie z.B. isotoner Kochsalzlösung (NaCl), und daraufhin erfolgt ein Entlüften des Schlauchsystems. Es besteht das Risiko, dass die Entlüftung des Leitungs-/Schlauchsystems fehlerhaft durchgeführt wird. Während der Messung besteht das Risiko, dass in die kristalloide Lösung in eine zu einem Messsensor führende Leitung eindringendes Fluid/Blut den Messvorgang beeinträchtigt oder unterbricht, was lebensgefährlich sein kann.

Auch nachteilig bei dieser Art Druckmessung sind Messfehler, die aufgrund von Gaseinschlüssen in der Wassersäule hervorgerufen werden. Auch kann die Druckmessung selbst meist nur verzögert durchgeführt werden, da eine Druckwelle zunächst durch das Wasser übertragen werden muss. Eine solche mittelbare Druckmessung erschwert dadurch z.B. eine Synchronisation einer Pumpe mit der arteriellen Druckkurve des Patienten.

Die deutsche Offenlegungsschrift DE 10 2005 063 410 A1 beschreibt einen Blutgefäßkatheter, bei welchem ein Blutdrucksensor an einem Gehäuse angeordnet ist, welches an eine Katheterröhre gekuppelt ist.

Die deutsche Offenlegungsschrift DE 10 2007 038 402 A1 beschreibt eine Vorrichtung zum Erfassen eines Drucks, bei welcher ein Sensorelement in eine Leitungswandstruktur getrennt von einem in der Leitung geführten Medium eingebettet ist.

Die europäische Patentschrift EP 0 328 558 B1 beschreibt einen elastischen Schlauch, auf dessen Innenseite eine Beschichtung aufgebracht ist, in welcher bei einer Verformung des Schlauchs ein Drucksignal gemessen werden kann.

Aus dem US Patent 6272930 B1 ist eine Vorrichtung zur Messung des Drucks einer Flüssigkeit bekannt, die eine Röhre durchfließt. Die Vorrichtung beinhaltet einen länglichen Körper mit einem ersten und einem zweiten Ende, einer axialen Bohrung, die zu den Enden hin geöffnet ist und die über eine innenliegende Wand definiert ist. Eine Aussparung ist zur axialen Bohrung hin geöffnet und liegt zwischen dessen beiden Enden. Beide Enden des Körpers besitzen Anschlussmittel zur Verbindung mit der Röhre. Ein Drucksensor ist abgedichtet in der Aussparung angeordnet, wobei die Oberfläche des Sensors, die dem Druck der Flüssigkeit ausgesetzt ist, eine Form hat, die bündig ist mit der Innenwand der axialen Bohrung im Bereich der Aussparung.

Aus der US Patentanmeldung US 2007/0261496 A1 ist eine biologische Flüssigkeitsvorrichtung bekannt, die einen Drucksensor beinhaltet, der an der Vorrichtung angeordnet ist. Der Drucksensor beinhaltet dabei einen kompressiblen Container, dessen Stauchung indikativ für den ausgeübten Druck ist.

Aus der europäischen Patentanmeldung EP 0685721 A2 ist ferner eine Vorrichtung zum Messen des Drucks eines Mediums bekannt. Die Vorrichtung besteht aus einem Drucksensor, der mit einem ersten Druckübertragungsteil in Kontakt steht und einem zweiten Druckübertragungsteil, das mit dem Medium, dessen Druck zu messen ist, in Verbindung steht. Das erste Druckübertragungsteil soll in seinem Randbereich derart durch translatorische Krafteinwirkung in axialer Richtung gegen das zweite Druckübertragungsteil gepresst werden, dass der Raum zwischen dem ersten und dem zweiten Druckübertragungsteil nach außen abgedichtet und möglichst klein ist, so dass sowohl Überdrücke als auch Unterdrücke im Medium messbar sind.

Es ist eine Aufgabe der vorliegenden Erfindung, eine medizintechnische Messvorrichtung, insbesondere Blutdruckmessvorrichtung, bereitzustellen, welche eine einfache Handhabung ermöglicht, insbesondere in Verbindung mit einer vergleichsweise exakten Messung. Die Aufgabe kann auch dadurch beschrieben werden, dass die medizintechnische Messvorrichtung einen einfachen Aufbau aufweisen soll und/oder ein Risiko von Verunreinigungen oder Lufteinschlüssen oder Leckagen klein halten kann.

Die Aufgabe wird gelöst durch eine medizintechnische Messvorrichtung gemäß Anspruch 1 zur Messung einer Eigenschaft eines Fluids, insbesondere zur Druckmessung, umfassend
- eine Leitung, insbesondere eine Schlauchleitung, die sich entlang einer Mittenlängsachse erstreckt und eingerichtet ist, ein Fluid, insbesondere Blut, innerhalb einer von einer Wandung umgrenzten, insbesondere zylindrischen Längskavität zu führen; und
- eine Sensoreinrichtung mit einem Drucksensor, die eingerichtet ist, eine Eigenschaft des in der Längskavität geführten Fluids zu messen; wobei bei der Messvorrichtung erfindungsgemäß vorgesehen ist, dass die Leitung eine in radialer Richtung in die Wandung eingebrachte Radialkavität aufweist, in welcher die Sensoreinrichtung zumindest teilweise angeordnet ist und derart in die Wandung integriert ist, dass der Sensor in Kommunikation mit dem Fluid in der Längskavität ist.

Die Anordnung des Sensors direkt in der Wandung liefert messtechnische Vorteile oder auch konstruktive Vorteile. Der Sensor kann problemlos an einer messtechnisch bevorzugten Position angeordnet werden, insbesondere in unmittelbarer Nähe zu einem Strömungspfad des Fluids, oder auch direkt im Strömungspfad. Insbesondere kann auch die Anzahl der Schnittstellen verringert werden, insbesondere auf nur eine einzige Schnittstelle in der Wandung. Die in die Wandung integrierte Anordnung des Sensors macht Zwischenstücke, Kupplungen oder sonstige Anschlüsse entbehrlich. Es sind z.B. keine Luer-Anschlüsse oder andere Zugänge zur Leitung erforderlich. Hierdurch kann das Risiko von Leckstellen oder von irgendwelchen unsterilen Schnittstellen deutlich vermindert werden. Bei den im Stand der Technik meist verwendeten Zwischenstücken oder Adaptern, insbesondere Luer-Anschlüssen, hingegen liegen immer mindestens zwei oder drei Schnittstellen vor, nämlich zum einen die Schnittstelle(n) zwischen dem Zwischenstück oder Adapter und der Leitung, und zum anderen die Schnittstelle zwischen dem Zwischenstück oder Adapter und dem Sensor. Es sollte demnach noch angemerkt werden, dass es sich bei einer Leitung im Sinne der Erfindung nämlich insbesondere nicht um ein Zwischenstück, einen Konnektor oder einen Adapter handelt. Zudem kann eine Fehlbedienung irgendwelcher Anschlüsse vermieden werden. Es kann z.B. vermieden werden, dass z.B. in einem extrakorporalen Leitungssystem oder Schlauchsystem (wie z.B. bei der Dialyse, bei der Herz- oder Lungenunterstützung oder bei kardiopulmonalem Bypass in der Herzchirurgie, oder in einem Katheter bzw. einer Schleuse, z.B. in der Kardiologie) in einem Abschnitt mit negativem Druck (insbesondere mit einem Druck kleiner als Atmosphärendruck) Luft im Bereich der Druckmess-Anordnung in das Leitungs-/Schlauchsystem bzw. den Katheter eingezogen wird, oder dass in einem Abschnitt mit positivem Druck (insbesondere dort wo Fluid/Blut in den Körper zurückgeführt wird) Fluid/Blut aus dem Kreislauf austritt.

Ein Priming-Vorgang speziell in Bezug auf den Sensor ist dabei nicht mehr erforderlich, was Zeit einsparen kann und die Druckmessung in bestimmten (z.B. lebensbedrohlichen) Situationen besonders nützlich machen kann. Zusätzliche zu entlüftende Zuleitungen oder Schläuche liegen nicht mehr vor. Mit anderen Worten: Die integrierte Anordnung des Sensors ermöglicht eine "inline"-Messung ohne zeitliche Verzögerung. Das blutführende Leitungs-/Schlauchsystem (einschließlich einem eventuell vorgesehenen Katheter bzw. Kanüle) kann dabei unabhängig von einer Druckmessung gefüllt und entlüftet werden.

Sensoren, insbesondere Drucksensoren, sind seit den letzten Jahren mit immer kleineren Abmessungen erhältlich. Dies ermöglicht nun eine vorteilhafte Anordnung des Sensors in unmittelbarer Nähe des zu messenden Fluids. Der integrierte Sensor kann direkt am Fluidstrom angeordnet werden, ohne den Fluidstrom zu beeinflussen. Dies ermöglicht auch eine besonders exakte (Druck-)Messung. Hierbei kann z.B. auch eine Integration in eine Wandung erfolgen, welche nur eine Wandstärke im Bereich von 1 mm bis 3mm aufweist.

Eine solche Anordnung des Sensors ermöglicht auch eine weitgehend beliebige Auswahl der Position des Sensors an irgendeinem Abschnitt oder irgendeiner Umfangsposition entlang der Leitung, insbesondere Schlauchleitung. Die Sensoreinrichtung oder der Sensor kann dabei einen Teil der Wandung bilden.

Als Messvorrichtung (insbesondere Druckmessvorrichtung) ist dabei bevorzugt eine Vorrichtung zu verstehen, welche ein bestimmtes Fluid in einem bestimmten Zustand, insbesondere in einem Strömungszustand, führen bzw. zu- oder ableiten kann und mindestens eine Eigenschaft, insbesondere den Druck des Fluids erfassen und wahlweise auch zumindest teilweise auswerten kann. Die Messvorrichtung kann bevorzugt zumindest den Druck und wahlweise auch weitere physikalische oder chemische Größen erfassen und auswerten. Eine solche Messvorrichtung kann z.B. für eine invasive Druckmessung verwendet werden, oder in Verbindung mit einem extrakorporalen Kreislauf, z.B. zum Nierenersatz, zur Herz-Lungen-Unterstützung oder zur Leberunterstützung. Speziell bei einer invasiven/implantierten Anordnung können sich dabei auch die Vorteile einer guten Dichtheit und/oder Sterilität ergeben. Mittels der Messvorrichtung können z.B. Vitalfunktionen eines Patienten überwacht werden, z.B. Herzmuskelkontraktionen (Hämodynamik), oder es kann ein durch extrakorporale Zirkulation bedingter Druckverlust gemessen werden.

Die Messvorrichtung kann dabei mehrere unterschiedliche Sensoren umfassen. Zusätzlich zum Sensor kann die Messvorrichtung weitere Komponenten wie z.B. eine Schutzkappe, eine Steckerbuchse (z.B. eine vierpolige Steckerbuchse) oder eine bevorzugt'wasserabweisende Membran aufweisen, wobei die Membran einen Schutz des Sensors vor externen Einflüssen sicherstellen kann. Der Sensor kann beispielsweise nach dem Differenz-Druck-Prinzip arbeiten. Die Messvorrichtung weist dann bevorzugt eine Verbindung zur Außenluft bzw. Umgebung auf. Die Membran kann auf einer Sensorabdeckung über dem Sensor angebracht sein und den Sensor schützen, insbesondere gebondete Elektronik-Anschlüsse des Sensors. Die Membran kann verhindern dass Medien (z.B. Blut, Wasser, Isopropanol) in Kontakt mit diesen Anschlüssen des Sensors gelangen. Die Membran ist bevorzugt beidseitig hydrophob, also in beide Richtungen, wobei Luft bevorzugt ungehindert durchgelassen werden kann.

Eine Eigenschaft, insbesondere ein Druck, kann dabei auch an mehreren Messpositionen gemessen werden, je nach medizinischer Anwendung, insbesondere ein Saugdruck an einer ersten Position vor einer Pumpe, ein Pumpendruck an einer zweiten Position nach der Pumpe, und ein weiterer an einer dritten Position, insbesondere ein Reperfusionsdruck nach einem Membranventilator. Mit anderen Worten: Die Messvorrichtung kann wahlweise auch eine Vielzahl von Sensoreinrichtungen oder zumindest eine Vielzahl von Sensoren aufweisen. Dementsprechend kann die Leitung (einschließlich eines eventuell vorhandenen Katheters oder Kanüle) auch eine Vielzahl von radialen Radialkavitäten aufweisen.

Als Fluid ist dabei bevorzugt eine Flüssigkeit zu verstehen, jedoch kann das Fluid auch ein Gas sein oder zumindest gasförmige Bestandteile aufweisen.

Als Leitung ist dabei bevorzugt jede Art von Leitung zu verstehen, welche in Verbindung mit einer medizinischen Versorgung, Diagnose oder Therapie genutzt werden kann, z.B. auch in Verbindung mit irgendwelchen Kathetern. Die Leitung kann Teil eines medizinischen Instruments oder Bestecks sein. Die insbesondere blutführende Leitung kann Teil eines so genannten Schlauchsets sein oder dieses Schlauchset bilden. Die (Schlauch-)Leitung kann dabei auch eine den Zugang zum Körper sicherstellende Kanüle umfassen oder abschnittsweise als Kanüle ausgebildet sein. Bevorzugt ist die Leitung flexibel, also elastisch formbar. Insbesondere kann die Leitung gekrümmt oder gebogen werden. Die Elastizität der Leitung wird dabei durch die Sensoreinrichtung nicht oder nicht spürbar beeinflusst. Der Durchmesser der Leitung kann weitgehend frei gewählt werden. Zweckdienlich sind insbesondere Innendurchmesser von z.B. 3/8" oder 1/4".

Die Wandung oder auch die gesamte Leitung kann aus einem flexiblen Kunststoffmaterial, insbesondere aus Polyvinylchlorid (PVC)-Material ausgeführt sein. Im einfachsten Fall handelt es sich bei der Leitung z.B. um einen in der Medizintechnik häufig eingesetzten PVC-Schlauch. Bevorzugt ist das Kunststoffmaterial ein hochreines, phthalatfreies Weich-PVC. Eine Wandstärke der Wandung und/oder Leitung liegt dabei z.B. im Bereich von 1mm bis 5mm, bevorzugt im Bereich von 1.2mm bis 3.5mm, weiter bevorzugt im Bereich von 1.5mm bis 3mm, insbesondere im Bereich von 1.6mm bis 2.4mm.

Als Sensoreinrichtung ist dabei bevorzugt eine Komponente der Messvorrichtung zu verstehen, mittels welcher ein Messsignal, insbesondere ein Drucksignal erfasst und entweder weiterverarbeitet oder zumindest weitergeleitet werden kann.

Als Sensor (insbesondere Drucksensor) ist dabei bevorzugt eine Komponente der Messvorrichtung zu verstehen, mittels welcher eine Eigenschaft eines Fluids durch ein Messsignal, insbesondere ein Drucksignal zumindest erfasst werden kann. Eine Eigenschaft oder ein Zustand des Fluids kann dabei z.B. durch eine physikalische oder chemische Größe erfasst werden. Eine Eigenschaft kann z.B. durch einen bestimmten Anteil einer gasförmigen Komponente beschrieben werden, z.B. einem Volumenanteil an CO2 oder O2. Der Sensor kann dabei z.B. zur Messung des Infusionsdrucks oder Injektionsdrucks in fluidführenden Medizinprodukten eingerichtet sein. Beispielsweise kann ein piezoresistiver Sensor verwendet werden. Wahlweise können auch Sensoren eingesetzt werden, welche auf einem oder mehreren der folgenden physikalischen Prinzipien oder Funktionsweisen basieren: z.B. piezoelektrisch, kapazitiv, induktiv, frequenzanalog, oder Sensor mit Hallelement, faseroptischer Sensor. Der Sensor kann dabei an seiner dem Blut zugewandten Fläche in die Kontur eines Innenlumens der Leitung so eingepasst sein, dass ein stufenfreier und lückenloser Übergang zwischen dem Sensor und dem Innenlumen sichergestellt ist, insbesondere um Gerinnung oder Hämolyse im Bereich des Sensors zu vermeiden.

Als Radialkavität ist dabei bevorzugt eine in radialer Richtung eingebrachte oder vorgesehene Ausnehmung, eine Bohrung, eine Aussparung oder auch ein ausgehöhlter Bereich oder Abschnitt oder Aufnahmevolumen zu verstehen. Die Radialkavität kann auch durch eine sich in radialer Richtung erstreckende Längskavität gebildet sein, welche nur von einer Seite der Wandung zugänglich ist. Die Radialkavität muss nicht notwendigerweise eine Durchführung oder ein Loch in der Wandung sein.

Als Öffnung ist dabei bevorzugt ein Durchlass oder eine Radialkavität zu verstehen, welche die Wandung vollständig durchbricht, also durchgehend durch die Wandung vorgesehen ist.

Als Ausnehmung ist dabei bevorzugt eine sich in radialer Richtung von einer Innenseite der Wandung, also einer Innenmantelfläche, erstreckende Kavität zu verstehen, die nicht notwendigerweise bis zu einer Außenmantelfläche der Wandung verlaufen muss. Mit anderen Worten: Die Ausnehmung ist nicht notwendigerweise ein Loch in der Wandung, sondern kann die Wandung auch nur abschnittsweise in radialer Richtung aushöhlen.

Als eine Anordnung "in Kommunikation mit" ist dabei bevorzugt eine Anordnung zu verstehen, bei welcher der Sensor in direktem Kontakt mit dem Fluid ist. Der Sensor kann im Fluidstrom oder an der Seite des Fluidstroms oder an der Seite eines Strömungspfades des Fluidstroms angeordnet werden.

Gemäß einem Ausführungsbeispiel ist die Messvorrichtung eingerichtet, die Sensoreinrichtung in einer einzigen (insbesondere geometrischen) Schnittstelle mit der Leitung zu verbinden, insbesondere direkt an einer Außenmantelfläche der Wandung zu befestigen/fixieren und dadurch zu positionieren. Hierdurch kann das Risiko von Leckagen gesenkt werden, insbesondere im Vergleich zu Adaptern oder Zwischenstücken, welche mehrere Schnittstellen aufweisen. Mit anderen Worten braucht die Messvorrichtung nur eine einzige Schnittstelle oder einen einzigen Befestigungspunkt oder Zugangspunkt aufzuweisen, über welche(n) der Sensor in die Leitung integrierbar ist.

Die Sensoreinrichtung kann an der einzigen Schnittstelle zwischen der Sensoreinrichtung und der Leitung in die Leitung integriert sein. Die Schnittstelle ist dabei bevorzugt allein durch die Radialkavität oder durch die Radialkavität und eine Außenmantelfläche der Wandung gebildet.

Als Schnittstelle ist dabei bevorzugt mindestens eine Fläche zu verstehen, an welcher die Sensoreinrichtung mit der Wandung verbunden werden kann. Die Schnittstelle kann dabei auch unterschiedliche Flächen umfassen, die jedoch bevorzugt im gleichen Bereich der Wandung angeordnet sind, insbesondere angrenzend aneinander.

Erfindungsgemäß ist der Sensor in einer Radialposition zwischen einer/der Außenmantelfläche und einer Innenmantelfläche der Wandung innerhalb der Wandung beabstandet von der Außenmantelfläche angeordnet, insbesondere in einem Abschnitt, welcher näher zur Innenmantelfläche als zur Außenmantelfläche angeordnet ist. Hierdurch kann der Sensor nahe am Strömungspfad des Fluids oder sogar im Strömungspfad angeordnet werden, was eine vergleichsweise exakte "inline"-Messung ermöglicht.

Gemäß einem Ausführungsbeispiel ist der Sensor oder ein radial nach innen vorstehendes freies Ende der Sensoreinrichtung zumindest annähernd bündig zu einer/der Innenmantelfläche der Wandung angeordnet. Hierdurch können günstige Strömungsverhältnisse sichergestellt werden. Durch Hinterschneidungen oder Kanten verursachte Verwirbelungen können weitgehend vermieden werden. Hierdurch kann auch ein Risiko von Blutgerinnseln oder Hämolyse verringert werden.

Der Sensor kann dabei in einer Radialposition angeordnet sein, welche zumindest annähernd einem Radialabstand einer Innenmantelfläche der Leitung zur Mittenlängsachse im Bereich der Radialkavität entspricht. Mit anderen Worten: Der Sensor kann in einer Radialposition angeordnet sein, welche durch einen Radialabstand zur Mittenlängsachse entsprechend dem halben Durchmesser, also dem Radius der Längskavität gekennzeichnet ist. Der Sensor kann die Längskavität in radialer Richtung begrenzen.

Gemäß einem Ausführungsbeispiel ist die Radialkavität eine Öffnung, insbesondere eine zylindrische Bohrung mit einheitlichem Durchmesser. Hierdurch kann ein besonders einfacher Aufbau sichergestellt werden. Eine Öffnung kann jederzeit nachträglich und auch an einer beliebigen Position auf einfache Weise in die Wandung eingebracht werden. Ferner kann der Sensor von außen in der Radialkavität positioniert werden.

Gemäß einem Ausführungsbeispiel ist eine Innenfläche der Radialkavität zylindrisch oder konisch oder weist einen polygonen Querschnitt auf. Die Radialkavität ist dabei nicht notwendigerweise rund oder kreisrund, sondern kann einen beliebigen Querschnitt aufweisen. Bei einer runden oder zylindrischen Geometrie kann die Radialkavität auf einfache Weise abgedichtet werden, insbesondere mittels einer Übermaß-Passung.

Gemäß einem Ausführungsbeispiel ist ein Durchmesser oder eine Ausdehnung eines Querschnitts der Radialkavität kleiner als ein damit geometrisch korrespondierender Abschnitt der Sensoreinrichtung, insbesondere auch des Sensors. Hierdurch kann die einzige Schnittstelle weitgehend unabhängig von irgendeiner stoffschlüssigen Verbindung abgedichtet werden. Eine stoffschlüssige Verbindung im Bereich der Innenfläche der Radialkavität ist nicht erforderlich. Dabei kann die Radialkavität zusammen mit der Sensoreinrichtung und/oder dem Sensor eine Übermaßpassung bilden, an welcher die Wandung fluiddicht abdichtbar ist. Mit anderen Worten: Eine korrespondierende Geometrie kann die Fluiddichtheit an der Radialkavität zwischen dem Sensor und der Wandung sicherstellen, selbst wenn an dieser Schnittstelle kein Haftmittel, Kleber oder dergleichen vorgesehen ist.

Bevorzugt ist die Radialkavität kreisrund. Dies erleichtert eine passgenaue Anordnung der Sensoreinrichtung in der Radialkavität, insbesondere unabhängig von einer bestimmten Verdrehposition.

Als Übermaßpassung ist dabei bevorzugt eine Schnittstelle zu verstehen, bei welcher eine der zu kuppelnden Komponenten ein bestimmtes Aufmaß aufweist, durch welches sichergestellt werden kann, dass die beiden zu kuppelnden Komponenten nur bei einer geometrischen Anpassung aufeinander miteinander verbunden werden können, und dass eine jedenfalls spielfreie und spaltfreie Verbindung sichergestellt ist.

Ein Durchmesser oder eine Ausdehnung eines Querschnitts der Radialkavität liegt dabei z.B. im Bereich von 1 mm bis 5mm, bevorzugt im Bereich von 2mm bis 4mm, weiter bevorzugt im Bereich von 2.5mm bis 3.5mm, insbesondere im Bereich von 3mm bis 3.3mm, speziell 3.15mm. Gemäß einer Variante weist die Sensoreinrichtung dann einen Radialabschnitt auf, welcher einen Durchmesser oder eine Ausdehnung im Bereich von 1mm bis 5mm aufweist, bevorzugt im Bereich von 2mm bis 4mm, weiter bevorzugt im Bereich von 2.5mm bis 3.5mm, insbesondere im Bereich von 3mm bis 3.3mm, speziell 3.1mm, wobei der Radialabschnitt eine Übermaßpassung, insbesondere eine Presspassung, mit einer Innenfläche der Radialkavität bildet. Hierdurch kann eine Abdichtung der Radialkavität erfolgen, insbesondere auch ohne irgendein Haftmittel im Bereich der Radialkavität.

Gemäß einem speziellen Ausführungsbeispiel weist die Wandung einen zumindest annähernd konstanten Durchmesser und/oder eine zumindest annähernd konstante Wandstärke auf. Hierdurch kann sichergestellt werden, dass der Sensor mittels der Sensoreinrichtung in einer vordefinierbaren Radialposition positioniert werden kann, und zwar unabhängig davon, an welcher Stelle der Wandung die Radialkavität eingebracht wird.

Gemäß einem speziellen Ausführungsbeispiel erstreckt sich der Sensor zumindest annähernd über das gesamte Querschnittsprofil der Radialkavität und weist zumindest annähernd dieselbe Ausdehnung auf wie ein Durchmesser oder eine Ausdehnung der Radialkavität. Hierdurch kann der gesamte Querschnitt der Radialkavität für die Messung, insbesondere Druckmessung genutzt werden. Mit anderen Worten: Die Radialkavität kann vergleichsweise klein ausgebildet sein. Insbesondere kann die Radialkavität einen Durchmesser aufweisen, welcher nur so groß oder nur leicht größer ist als für die Messung erforderlich ist.

Gemäß einem speziellen Ausführungsbeispiel weist der Sensor und/oder die Sensoreinrichtung eine Stirnfläche auf, welche radial bündig zu einer Innenmantelfläche der Leitung angeordnet ist, wobei die Stirnfläche die Wandung weiterbildet, insbesondere über die gesamte Querschnittsfläche der Radialkavität. Hierdurch kann ein Übergang zwischen der Innenmantelfläche und der Radialkavität gebildet werden, so dass Hinterschneidungen oder Kanten und damit möglicherweise einhergehende Verwirbelungen oder Toträume weitgehend vermieden werden können. Es sollte ebenfalls erwähnt werden, dass der Sensor nicht in den Leitungsquerschnitt hineinragt. Gemäß einer speziellen Variante ist die Stirnfläche geometrisch korrespondierend zur Geometrie der Innenmantelfläche ausgebildet, insbesondere konkav gekrümmt. Hierdurch kann eine formoptimierte Integration erfolgen. Für den Blutstrom ist der Sensor dabei gar nicht "sichtbar". Ein Risiko von Verwirbelungen kann so gut wie ausgeschlossen werden.

Gemäß einem Ausführungsbeispiel ist die Sensoreinrichtung eingerichtet, den Sensor in einer vordefinierten Radialposition in der Radialkavität zu positionieren, insbesondere indem die Sensoreinrichtung in einem dafür eingerichteten Abschnitt geometrisch korrespondierend zu einer Außenmantelfläche der Leitung/Wandung ausgebildet ist. Die Sensoreinrichtung kann dafür zumindest abschnittsweise derart geometrisch ausgebildet sein, dass die Sensoreinrichtung in einer vorbestimmten Relativposition direkt an der Wandung positioniert werden kann. Hierdurch kann die Radialposition des Sensors durch geometrisches Ausgestalten der Sensoreinrichtung vorgegeben werden. Beispielsweise kann dadurch auch die Montage des Sensors in der "richtigen" Position vereinfacht werden. Die Sensoreinrichtung braucht beispielsweise nur auf der Außenmantelfläche angeordnet werden, insbesondere in einer Längsausrichtung entsprechend der Mittenlängsachse.

Gemäß einem Ausführungsbeispiel weist die Sensoreinrichtung einen Anlageabschnitt auf, welcher zumindest im Bereich der Radialkavität, insbesondere umlaufend um die Radialkavität, geometrisch korrespondierend zu einer/der Außenmantelfläche der Wandung ausgebildet ist. Hierdurch kann die Sensoreinrichtung auf einfache und robuste Weise mit der Leitung verbunden werden, und kann insbesondere im Bereich der Radialkavität umlaufend um die Radialkavität an die Leitung angeklebt werden. Der Anlageabschnitt weist z.B. eine konkave Kontur auf, welche geometrisch korrespondierend zu einer konvexen Kontur der Leitung oder Wandung ausgebildet ist.

Der Anlageabschnitt ist dabei bevorzugt eingerichtet, die Radialposition des Sensors zu definieren, nämlich über die Außenmantelfläche der Wandung. Mit anderen Worten: Eine radiale Erstreckung einer Sensoraufnahme oder eines Radialabschnittes der Sensoreinrichtung ist derart auf die Geometrie des Anlageabschnittes abgestimmt, dass der Sensor bei flächigem Anlegen des Anlageabschnitts auf die Außenmantelfläche der Wandung in einer vorbestimmten Radialposition angeordnet ist, insbesondere innerhalb der Radialkavität.

Als Anlageabschnitt ist dabei bevorzugt ein flächiger Abschnitt zu verstehen, an welchem die Sensoreinrichtung an der Leitung zur Anlage gebracht werden kann. Bevorzugt ist der Anlageabschnitt auch formstabil, also elastisch oder plastisch nicht verformbar, so dass eine Relativbewegung zwischen der Leitung und dem Anlageabschnitt im Bereich der Radialkavität vermieden werden kann. Dies kann eine dauerhaft, sichere Verbindung zwischen diesen beiden Komponenten sicherstellen.
In einer vorteilhaften Ausbildung der Erfindung ist die Sensoreinrichtung teilweise in der Radialkavität in der Wandung angeordnet und derart in die Wandung integriert, dass der Sensor in Kommunikation mit dem Fluid ist, und dass der Anlageabschnitt der Sensoreinrichtung umlaufend um die Radialkavität und geometrisch korrespondierend zu einer/der Außenmantelfläche der Wandung ausgebildet ist.

Die Sensoreinrichtung weist also einen Teil auf, nämlich den Sensor oder zumindest einen Teil des Sensors, der sich in der Radialkavität in der Wandung der Leitung befindet und einen anderen Teil, der außen an der Leitung angeordnet ist und über den flächigen Abschnitt an der Außenwand der Leitung zur Anlage gebracht werden kann. Durch die stoffschlüssige Verbindung des flächigen Abschnitts bzw. des Anlageabschnitts der Sensoreinrichtung um den Sensor in der Radialkavität herum, wird eine optimale Abdichtung der Leitung nach außen hin gewährleistet. Durch das Hineinragen des Sensors in die Radialkavität wird neben der stoffschlüssigen Festlegung der Sensoreinrichtung an der Leitung auch noch eine mechanische Festlegung insbesondere im Hinblick auf Zugkräfte in Richtung der Leitungslängserstreckung gewährleistet.

Gemäß einem Ausführungsbeispiel weist die Sensoreinrichtung einen Anlageabschnitt auf, welcher zumindest im Bereich der Radialkavität, insbesondere umlaufend um die Radialkavität, stoffschlüssig mit einer/der Außenmantelfläche der Leitung oder Wandung verbunden ist, insbesondere mittels eines Haftmittels. Hierdurch kann die Sensoreinrichtung auf robuste Weise an der Leitung fixiert werden. Die stoffschlüssige Verbindung im Bereich der Außenmantelfläche hat auch den Vorteil, dass irgendein Haftmittel, insbesondere Kleber, nicht notwendigerweise an einer Innenfläche der Radialkavität vorgesehen sein muss. Hierdurch kann vermieden werden, dass das Fluid, insbesondere Blut, in Kontakt mit dem Haftmittel gelangt. Bevorzugt ist das Haftmittel flexibel und feuchtigkeitsbeständig. Bevorzugt ist das Haftmittel eingerichtet, eine dauerhafte Haftverbindung zu Kunststoff, insbesondere PVC sicherzustellen. Bevorzugt ist das Haftmittel ein aushärtender Kleber.

Beispielsweise ist der Anlageabschnitt im Bereich der Radialkavität umlaufend um die Radialkavität mit der Außenmantelfläche mittels des Haftmittels verbunden, wobei die Sensoreinrichtung direkt an einer Innenfläche der Radialkavität, ohne Haftmittel, passgenau und fluiddicht zur Anlage gebracht ist. Hierdurch kann zum einen eine verhältnismäßig robuste, belastbare flächige Verbindung zwischen der Leitung und dem Anlageabschnitt sichergestellt werden. Zum anderen kann effektiv vermieden werden, dass die Leitung relativ zur Sensoreinrichtung verdreht wird.

Es sollte angemerkt werden, dass eine ausschließliche Anordnung des Sensors und der Sensoreinrichtung in der Radialkavität einer flexiblen bzw. elastischen Leitung problematisch sein kann, da es leicht zu Undichtigkeiten kommen kann, wenn die flexible oder elastische Leitung sich bewegt oder bewegt wird, während der als steifes Bauteil vorliegende Sensor, bzw. die Sensoreinrichtung, starr in der Radialkavität angeordnet ist und diese Bewegung nicht mitvollziehen kann. Es könnte daher zu Leckagen in der stoffschlüssigen Anbindung der Sensoreinrichtung in der Kavität zur Wandung der Leitung kommen.

Gemäß einem Ausführungsbeispiel weist die Sensoreinrichtung einen Radialabschnitt auf, in welchem der Sensor angeordnet ist, wobei der Radialabschnitt in radialer Richtung bevorzugt eine Erstreckung aufweist, die bevorzugt größer ist als die Hälfte einer Wandstärke der Wandung in einem Abschnitt, in welchem die Radialkavität angeordnet ist. Hierdurch kann der Sensor mittels des Radialabschnittes in einer vordefinierten Position in der Radialkavität angeordnet werden, insbesondere auf vergleichsweise exakte Weise in einer exakt vorgebbaren Radialposition, z.B. genau auf Höhe der Innenmantelfläche der Wandung. Gemäß einer Variante kann die Radialerstreckung zumindest annähernd der Wandstärke entsprechen, so dass der Sensor nahezu im Strömungspfad des Fluids positioniert werden kann, also zumindest annähernd in demselben Radialabstand von einer Mittenlängsachse der Leitung wie die Innenmantelfläche der Wandung. Der Radialabschnitt ist dabei an die Wandstärke anpassbar. Mit anderen Worten: Die Position des Sensors ist durch eine vorbestimmte Radialerstreckung des Radialabschnitts vorgebbar/definierbar.

Als Radialabschnitt ist dabei bevorzugt ein sich in radialer Richtung erstreckender Abschnitt zu verstehen, welcher eingerichtet ist, mit der Radialkavität gekuppelt zu werden und welcher im Querschnitt geometrisch korrespondierend zum Querschnittsprofil der Radialkavität ausgebildet ist. Der Radialabschnitt kann dabei eine Sensoraufnahme umfassen, welche eingerichtet ist, den Sensor an der Sensoreinrichtung zu halten oder zu befestigen. Als Sensoraufnahme ist dabei bevorzugt ein Teil der Sensoreinrichtung zu verstehen, an welchem der Sensor (oder ein ganz bestimmter Typ Sensor) an der Sensoreinrichtung positioniert werden kann, sofern der Sensor nicht in die Sensoreinrichtung integriert ist oder durch die Sensoreinrichtung gebildet wird. Die Sensoraufnahme kann dabei z.B. auch elektrische Kontakte aufweisen, mittels welchen der Sensor in elektrischer Verbindung mit einem Kabel oder irgendeiner Kommunikationsschnittstelle oder Energieversorgung steht. Der Radialabschnitt kann dabei auch zumindest abschnittsweise durch die Sensoraufnahme gebildet sein.

Gemäß einem Ausführungsbeispiel ist die Sensoreinrichtung zumindest teilweise oder zumindest ein/der Radialabschnitt der Sensoreinrichtung aus einem Kunststoffmaterial ausgebildet. Hierdurch kann eine Schnittstelle zur Wandung bereitgestellt werden, welche auch effektiv abgedichtet werden kann, insbesondere bei PVC-Schläuchen. Auch kann ein Anlageabschnitt der Sensoreinrichtung dabei effektiv mit der Wandung verbunden werden (vorteilhafte Materialpaarung).

Gemäß einem Ausführungsbeispiel ist die Messvorrichtung eine für den einmaligen Gebrauch vorgesehene Einwegvorrichtung, wobei die Sensoreinrichtung bevorzugt eine Kupplungsstelle für eine Kommunikation und/oder Energieversorgung aufweist, insbesondere für eine drahtgebundene Übertragung über ein Kabel oder für eine drahtlose Übertragung. Die Integration des Sensors in die Wandung ermöglicht eine einfach aufgebaute Messvorrichtung, bei welcher z.B. lediglich ein Kabel oder ein Stick entfernt werden muss, bevor die Messvorrichtung entsorgt wird.

Gemäß einem Ausführungsbeispiel kann ein Messsystem für extrakorporale Zirkulation mit mindestens zwei erfindungsgemäßen Messvorrichtungen gebildet werden, wobei eine der Messvorrichtungen eine zuführende Leitung aufweist und eine andere der Messvorrichtungen eine abführende Leitung aufweist. Hierdurch kann in einer Kreislaufführung sowohl eine Eigenschaft, insbesondere ein Druck eines abgeführten Fluids als auch eines zugeführten Fluids gemessen werden. Dabei kann in der abführenden Leitung insbesondere das Risiko gesenkt werden, dass (speziell an irgendwelchen Luer-Anschlüssen) Luft in die Leitung eingezogen wird. In der zuführenden Leitung kann insbesondere das Risiko gesenkt werden, dass (speziell an irgendwelchen Luer-Anschlüssen) das Fluid (insbesondere Blut) bei einem Überdruck aus der Leitung gepresst wird oder herausspritzt. Mit anderen Worten: Die Integration der Sensoren in die Wandung liefert den Vorteil, dass Leckagen weitgehend ausgeschlossen werden können. Die Anzahl der Schnittstellen kann reduziert werden, insbesondere auf eine einzige Schnittstelle je Leitung.

Die Aufgabe wird auch gelöst durch ein Verfahren gemäß Anspruch 14 zum Herstellen einer medizintechnischen Messvorrichtung, insbesondere einer erfindungsgemäßen medizintechnischen Messvorrichtung, umfassend die Schritte:
- Bereitstellen einer Leitung mit einer Wandung, insbesondere einer flexiblen, biegsamen Schlauchleitung;
- Anordnen eines Sensors an der Leitung;
bei welchem erfindungsgemäß vorgesehen ist, dass eine Radialkavität in radialer Richtung in die Wandung eingebracht wird und der Sensor in der Radialkavität angeordnet wird, insbesondere an einer Radialposition zwischen einer Außenmantelfläche und einer Innenmantelfläche der Wandung beabstandet von der Außenmantelfläche. Hierdurch ergeben sich insbesondere die in Verbindung mit der Messvorrichtung erläuterten Vorteile.

Gemäß einem Ausführungsbeispiel des Verfahrens wird die Messvorrichtung mit einer Außenmantelfläche der Wandung stoffschlüssig verbunden und/oder der Sensor in der Radialkavität in einer Übermaßpassung fluiddicht in der Wandung positioniert. Hierdurch kann eine Messvorrichtung bereitgestellt werden, die eine einzige, vergleichsweise robuste und belastbare Schnittstelle aufweist, die mit guter Sicherheit fluiddicht abgedichtet werden kann. Bevorzugt erfolgt die Abdichtung allein in der Radialkavität, und die eindeutige relative Position kann bevorzugt durch die stoffschlüssige Verbindung sichergestellt werden. Eine stoffschlüssige Verbindung kann dabei die Robustheit erhöhen.

Die Aufgabe wird auch gelöst durch ein Verfahren gemäß Anspruch 18 zur Messung mittels einer medizintechnischen Messvorrichtung, insbesondere zur Druckmessung mittels einer erfindungsgemäßen medizintechnischen Messvorrichtung, umfassend die Schritte:
- Führen eines Fluids, insbesondere Blut, durch eine Leitung innerhalb einer von einer Wandung umgrenzten Längskavität, insbesondere durch eine Schlauchleitung;
- Messen einer Eigenschaft des Fluids mittels eines Sensor der Messvorrichtung; bei welchem erfindungsgemäß vorgesehen ist, dass der Sensor in einer in die Wandung eingebrachten Radialkavität positioniert ist und die Eigenschaft in der Radialkavität gemessen wird, insbesondere an einer Radialposition zwischen einer Außenmantelfläche und einer Innenmantelfläche der Wandung beabstandet von der Außenmantelfläche. Die Messung, insbesondere Druckmessung kann "inline" innerhalb der Wandung erfolgen. Die integrierte Anordnung des Sensors ermöglicht eine "inline"-Messung ohne zeitliche Verzögerung.

In den nachfolgenden Zeichnungsfiguren wird die Erfindung anhand von Ausführungsbeispielen näher erläutert. Es zeigen:
Figur 1 in schematischer Darstellung in perspektivischer Ansicht eine Leitung einer medizintechnischen Messvorrichtung gemäß einem Ausführungsbeispiel der Erfindung;
Figur 2 in schematischer Darstellung in perspektivischer Ansicht eine Sensoreinrichtung einer medizintechnischen Messvorrichtung gemäß einem Ausführungsbeispiel der Erfindung in einer Anordnung an der in der Fig. 1 gezeigten Leitung;
Figur 3 in schematischer Darstellung in einer Schnittansicht eine medizintechnische Messvorrichtung gemäß einem Ausführungsbeispiel der Erfindung;
Figuren 4 und 5 jeweils in schematischer Darstellung in unterschiedlichen perspektivischen, geschnittenen Ansichten die in der Figur 3 gezeigte medizintechnische Messvorrichtung; und
Figur 6 in schematischer Darstellung in einer Seitenansicht eine Sensoreinrichtung einer medizintechnischen Messvorrichtung gemäß einem Ausführungsbeispiel der Erfindung.

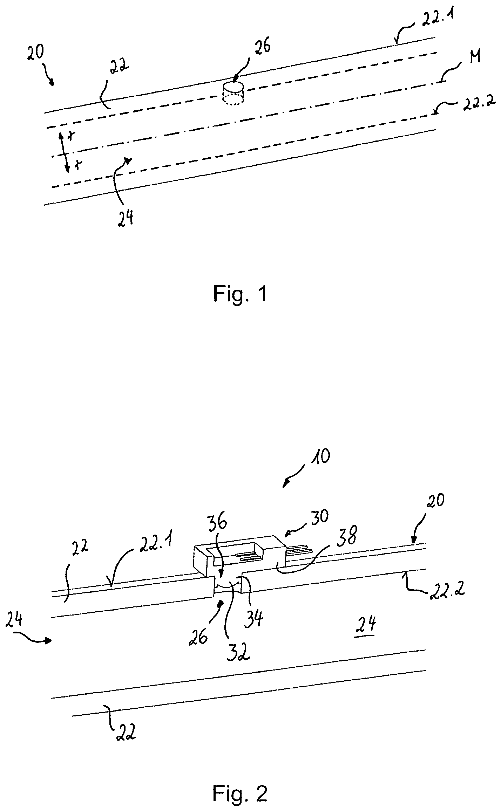

Im Zusammenhang mit der Beschreibung der folgenden Figuren wird bei Bezugszeichen, die nicht explizit erläutert werden, auf das Ausführungsbeispiel der Figur 1 verwiesen.

In der Figur 1 ist eine Leitung 20 in Form einer Schlauchleitung gezeigt, welche eine Wandung 22 aufweist. Die Leitung 20 kann aus einem elastischen Material ausgebildet sein und flexibel bzw. biegsam sein. Die Wandung 22 weist eine Außenmantelfläche 22.1 und eine Innenmantelfläche 22.2 auf. Die Wandung 22 bzw. die Innenmantelfläche 22.2 umgrenzt eine Längskavität 24, in welcher ein Fluid, insbesondere Blut, geführt werden kann. Die Leitung 20 erstreckt sich in Längsrichtung entlang einer Mittenlängsachse M. In radialer Richtung r ist eine Radialkavität 26 in die Wandung 22 eingebracht. Die Radialkavität 26 ist eine Radialöffnung in Form eines Lochs, welches sich von der Außenmantelfläche 22.1 zur Innenmantelfläche 22.2 erstreckt und einen Durchlass bildet. In diesen Durchlass kann nun ein Sensor oder eine Sensoreinrichtung positioniert werden, und eine Abdichtung kann dabei mittels des Sensors oder der Sensoreinrichtung erfolgen, wie im Zusammenhang mit den folgenden Figuren erläutert wird.

In der Figur 2 ist eine medizintechnische Messvorrichtung 10 gezeigt, welche die in Figur 1 beschriebene Leitung 20 und eine Sensoreinrichtung 30 aufweist. In oder an der Sensoreinrichtung 30 ist ein Sensor 32 angeordnet. Der Sensor 32 kann als eine Komponente der Sensoreinrichtung 30 aufgefasst werden. Der Sensor 32 bzw. zumindest teilweise auch die Sensoreinrichtung 30 ist in der Radialkavität 26 angeordnet, wobei der Sensor 32 zwischen der Außenmantelfläche 22.1 und der Innenmantelfläche 22.2 positioniert ist. Die Sensoreinrichtung 30 weist einen sich in radialer Richtung erstreckenden Radialabschnitt 36 und einen sich in Längsrichtung erstreckenden Anlageabschnitt 38. Der Radialabschnitt 36 umfasst eine Sensoraufnahme 34, in welcher der Sensor 32 angeordnet ist. Die Sensoraufnahme 34 und/oder der Sensor 32 sind geometrisch korrespondierend zur Radialkavität 26 ausgebildet. Der Anlageabschnitt 38 kommt an der Außenmantelfläche 22.1 zur Anlage, insbesondere flächig umlaufend um die Radialkavität 26 herum.

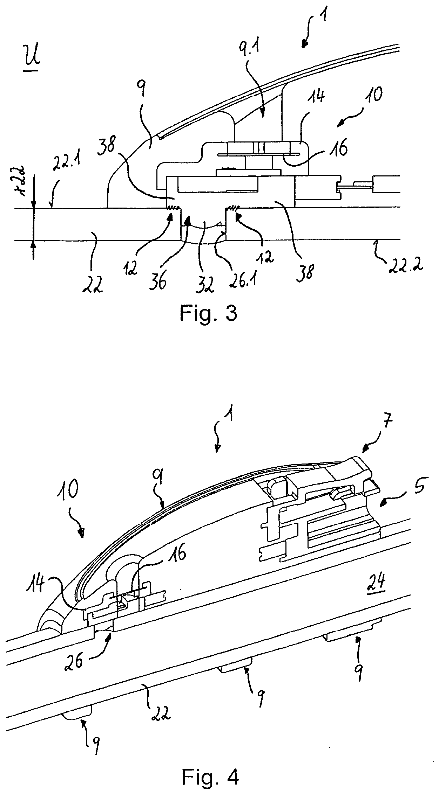

Die Figur 3 zeigt ein medizintechnisches Messsystem 1, welches unter anderem die in der Figur 2 dargestellte medizintechnische Messvorrichtung 10 sowie eine Umspritzung 9 mit einem Durchlass 9.1 umfasst, wobei der Sensor 32 über den Durchlass 9.1 in Kommunikation mit der Umgebung U ist. Der Sensor 32 ist durch eine Abdeckung 14 abgedeckt und durch eine luftdurchlässige, wasserabweisende Membran 16 geschützt. In der Figur 3 ist im Detail gezeigt, dass der Radialabschnitt 36 passgenau flächig an einer Innenfläche 26.1 der Radialkavität 26 anliegt, insbesondere umlaufend, um eine Abdichtung der Längskavität sicherstellen zu können. Der Anlageabschnitt 38 hingegen kontaktiert nur die Außenmantelfläche 22.1, und zwar abschnittsweise direkt und/oder wahlweise (wie angedeutet) mittelbar über ein Haftmittel 12. Das Haftmittel 12 ist vorzugsweise vollständig umlaufend um die Radialkavität 26 herum auf der Außenmantelfläche 22.1 und/oder dem Anlageabschnitt vorgesehen. Das Haftmittel 12 ist jedoch nicht an der Innenfläche 26.1 vorgesehen, insbesondere um Kontakt mit dem in der Längskavität geführten Fluid vermeiden zu können.

Die Radialposition des Sensors 32 sei anhand der Figur 3 nur beispielhaft erläutert. Die Radialposition des Sensors 32 kann von der gezeigten Position abweichen. Insbesondere kann der Sensor 32 weiter innen näher zur Innenmantelfläche 22.2 angeordnet sein, was in vielen Anwendungsfällen Vorteile liefern kann, z.B. Vorteile einer direkten "inline"-Messung im Strömungspfad, also zumindest annähernd ohne den Einfluss von irgendwelchen Strömungsverwirbelungen an irgendwelchen Hinterschneidungen oder Kanten.

Die Wandung 22 weist eine Wandstärke r22 auf, die z.B. im Bereich von 1 mm bis 5mm liegt, insbesondere im Bereich von 1.6mm bis 2.4mm. Die Radialposition des Sensors 32 ist zumindest annähernd mittig in Bezug auf die Außen- und Innenmantelflächen 22.1, 22.2. Der Radialabschnitt 36 kann jedoch eine größere Radialerstreckung aufweisen als dargestellt. Insbesondere kann der Radialabschnitt 36 eine Radialerstreckung aufweisen, die im Bereich der Wandstärke r22 liegt.

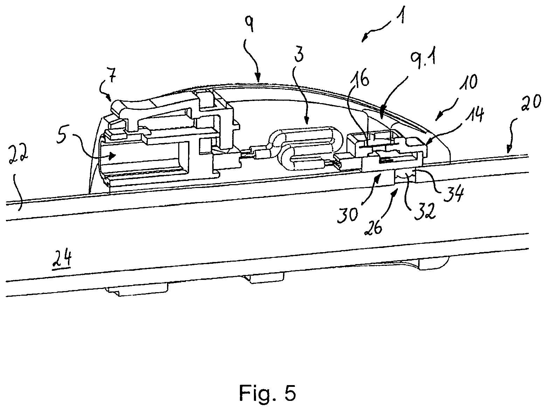

In den Figuren 4 und 5 sind weitere Komponenten des Messsystems 1 gezeigt, insbesondere eine Steckerbuchse 5, welche eine Kupplungsstelle für eine Kommunikation und/oder Energieversorgung bereitstellt, und ein Taster 7 zum manuellen Betätigen des Messsystems 1. Die Steckerbuchse 5 ist mittels eines Adapterkabels 3, welches in der Umspritzung 9 angeordnet ist, mit der Sensoreinrichtung 30 und dem Sensor 32 verbunden.

Die in der Figur 5 gezeigte Steckerbuchse 5 ist eingerichtet, entweder einen Stecker in Verbindung mit einem (externen) Kabel oder eine Art "Stick" oder Modul aufzunehmen. Der Stick kann eine drahtlose Kommunikation sicherstellen, z.B. über WLAN, Funk, bluetooth. Der Stick kann auch eine Energieversorgung aufweisen, z.B. einen Akku. Die Steckerbuchse 5 kann dabei für beide Varianten dieselbe Form aufweisen, so dass ein Benutzer jeweils entscheiden kann, ob eine drahtgebundene Energieversorgung und Kommunikation erwünscht ist, oder ob die Kommunikation drahtlos und die Energieversorgung über den Stick, z.B. mittels im Stick integrierten Batterien, erfolgen soll. Sowohl das Kabel als auch der Stick können dabei mehrfach verwendet werden. Mit anderen Worten: das medizintechnische Messsystem 1 oder die medizintechnische Messvorrichtung 10 können für den Einmalgebrauch ("disposable") vorgesehen sein, und vor dem Entsorgen kann das Kabel oder der Stick dann von der Steckerbuchse entkoppelt werden.

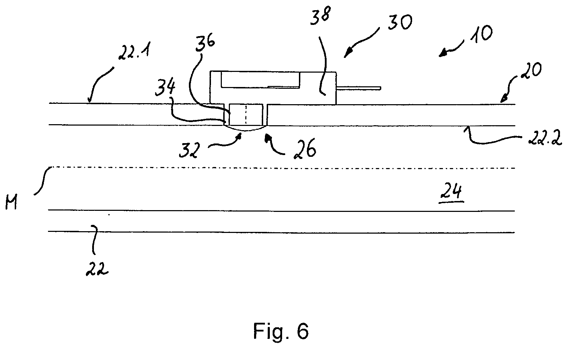

In der Figur 6 ist eine medizintechnische Messvorrichtung 10 gezeigt, welche vergleichbar zu der in der Figur 2 gezeigten Sensoreinrichtung 30 aufgebaut ist. Bei dieser Sensoreinrichtung 30 ist die Erstreckung des Radialabschnitts 36 derart auf die Wandstärke der Wandung 22 abgestimmt, dass der Sensor 32 bündig mit der Innenmantelfläche 22.2 angeordnet ist.

### Bezugszeichenliste

- 1: medizintechnisches Messsystem, insbesondere Druckmesssystem
- 3: Adapterkabel
- 5: Steckerbuchse
- 7: Taster
- 9: Umspritzung
- 9.1: Durchlass in Umspritzung
- 10: medizintechnische Messvorrichtung
- 12: Haftmittel, insbesondere Kleber
- 14: Abdeckung, insbesondere Schutzkappe
- 16: Membran
- 20: Leitung, insbesondere Schlauchleitung
- 22: Wandung
- 22.1: Außenmantelfläche der Wandung
- 22.2: Innenmantelfläche der Wandung
- 24: Längskavität
- 26: Radialkavität, insbesondere Radialöffnung
- 26.1: Innenmantelfläche der Radialkavität
- 30: Sensoreinrichtung, insbesondere Drucksensoreinrichtung
- 32: Sensor, insbesondere Drucksensor
- 34: Sensoraufnahme
- 36: Radialabschnitt
- 38: Anlageabschnitt

- M: Mittenlängsachse
- r: radiale Richtung
- r22: Wandstärke der Wandung

## Patentansprüche

1. Medizintechnische Messvorrichtung (10) zur Messung einer Eigenschaft eines Fluids, nämlich zur Druckmessung, umfassend
- eine Leitung (20), die sich entlang einer Mittenlängsachse (M) erstreckt und eingerichtet ist, ein Fluid, insbesondere Blut, innerhalb einer von einer Wandung (22) umgrenzten Längskavität (24) zu führen;
- eine Sensoreinrichtung (30) mit einem Drucksensor (32), die eingerichtet ist, eine Eigenschaft des in der Längskavität (24) geführten Fluids zu messen;
wobei die Sensoreinrichtung (30) einen Anlageabschnitt (38), der an der Außenmantelfläche (22.1) der Wandung (22) zur Anlage kommt, und einen Radialabschnitt (36) aufweist,
wobei die Leitung (20) als flexible Schlauchleitung ausgebildet ist und eine in radialer Richtung in die Wandung (22) eingebrachte Radialkavität (26) aufweist, in welcher die Sensoreinrichtung (30) zumindest teilweise angeordnet ist und derart in die Wandung (22) integriert ist, dass der Drucksensor (32) in Kommunikation mit dem Fluid ist, **dadurch gekennzeichnet, dass** die Drucksensor-Oberfläche innerhalb der Wandung (22) positioniert ist.

2. Messvorrichtung (10) nach Anspruch 1, wobei die Messvorrichtung (10) eingerichtet ist, die Sensoreinrichtung (30) in einer einzigen Schnittstelle mit der Leitung (20) zu verbinden, insbesondere direkt an einer Außenmantelfläche (22.1) der Wandung (22) zu befestigen.

3. Messvorrichtung (10) nach einem der vorhergehenden Ansprüche, wobei der Sensor (32) in einer Radialposition zwischen einer/der Außenmantelfläche (22.1) und einer Innenmantelfläche (22.2) der Wandung innerhalb der Wandung beabstandet von der Außenmantelfläche (22.1) angeordnet ist, insbesondere in einem Abschnitt, welcher näher zur Innenmantelfläche (22.2) als zur Außenmantelfläche (22.1) angeordnet ist.

4. Messvorrichtung (10) nach einem der vorhergehenden Ansprüche, wobei die Radialkavität (26) eine Öffnung ist, insbesondere eine zylindrische Bohrung mit einheitlichem Durchmesser.

5. Messvorrichtung nach einem der vorhergehenden Ansprüche, wobei ein Durchmesser oder eine Ausdehnung eines Querschnitts der Radialkavität (26) kleiner ist als ein damit geometrisch korrespondierender Abschnitt der Sensoreinrichtung (30), wobei die Radialkavität zusammen mit der Sensoreinrichtung und/oder dem Sensor (32) eine Übermaßpassung bildet, an welcher die Wandung (22) fluiddicht abdichtbar ist.

6. Messvorrichtung (10) nach einem der vorhergehenden Ansprüche, wobei die Sensoreinrichtung (30) eingerichtet ist, den Drucksensor (32) in einer vordefinierten Radialposition in der Radialkavität (26) zu positionieren.

7. Messvorrichtung (10) nach einem der vorhergehenden Ansprüche, wobei die Sensoreinrichtung (30) einen Anlageabschnitt (38) aufweist, welcher zumindest im Bereich der Radialkavität (26), insbesondere umlaufend um die Radialkavität, geometrisch korrespondierend zu einer/der Außenmantelfläche (22.1) der Wandung (22) ausgebildet ist.

8. Messvorrichtung (10) nach Anspruch 7, wobei die Sensoreinrichtung (30) teilweise in der Radialkavität (26) in der Wandung (22) angeordnet ist und derart in die Wandung (22) integriert ist, dass der Drucksensor (32) in Kommunikation mit dem Fluid ist, und dass der Anlageabschnitt (38) der Sensoreinrichtung (30) umlaufend um die Radialkavität (26) und geometrisch korrespondierend zu einer/der Außenmantelfläche (22.1) der Wandung (22) ausgebildet ist.

9. Messvorrichtung (10) nach einem der vorhergehenden Ansprüche, wobei ein/der Anlageabschnitt (38) der Sensoreinrichtung (30) im Bereich der Radialkavität (26), insbesondere umlaufend um die Radialkavität, stoffschlüssig mit einer/der Außenmantelfläche (22.1) der Wandung (22) verbunden ist, insbesondere mittels eines Haftmittels (12).

10. Messvorrichtung (10) nach einem der vorhergehenden Ansprüche, wobei die Sensoreinrichtung (30) einen Radialabschnitt (36) aufweist, in welchem der Drucksensor (32) angeordnet ist, wobei der Radialabschnitt in radialer Richtung bevorzugt eine Erstreckung aufweist, die bevorzugt größer ist als die Hälfte einer Wandstärke (r22) der Wandung (22) in einem Abschnitt, in welchem die Radialkavität (26) angeordnet ist.

11. Messvorrichtung (10) nach einem der vorhergehenden Ansprüche, wobei die Sensoreinrichtung (30) zumindest teilweise, insbesondere zumindest ein/der Radialabschnitt (36) der Sensoreinrichtung, aus einem Kunststoffmaterial ausgebildet ist.

12. Messvorrichtung (10) nach einem der vorhergehenden Ansprüche, **dadurch gekennzeichnet, dass** sie eine für den einmaligen Gebrauch vorgesehene Einwegvorrichtung ist, wobei die Sensoreinrichtung (30) bevorzugt eine Kupplungsstelle für eine Kommunikation und/oder Energieversorgung aufweist, insbesondere für eine drahtgebundene Übertragung über ein Kabel oder für eine drahtlose Übertragung.

13. Messsystem (1) für extrakorporale Zirkulation, mit mindestens zwei Messvorrichtungen (10) nach einem der vorhergehenden Ansprüche, wobei eine der Messvorrichtungen eine zuführende Leitung (20) aufweist und eine andere der Messvorrichtungen eine abführende Leitung (20) aufweist.

14. Verfahren zum Herstellen einer medizintechnischen Messvorrichtung (10), nach einem der vorhergehenden Ansprüche, umfassend die Schritte:
- Bereitstellen einer Leitung (20) mit einer Wandung (22), in Form einer flexiblen, biegsamen Schlauchleitung;
- Bereitstellen einer Sensoreinrichtung (30) mit einem Anlageabschnitt (38) und einem Radialabschnitt (36), umfassend eine Sensoraufnahme (34), in welcher ein Drucksensor (32) angeordnet ist;
- Anordnen eines Drucksensors (32) an der Leitung (20);
wobei eine Radialkavität (26) in radialer Richtung in die Wandung (22) eingebracht wird und der Drucksensor (32) in der Radialkavität (26) angeordnet wird, **dadurch gekennzeichnet, dass** die Sensoroberfläche des Drucksensors (32) in der Radialkavität (26) positioniert wird und die Sensoreinrichtung (30) mit einem Anlageabschnitt (38) an der Außenmantelfläche (22.1) der Wandung (22) zur Anlage kommt.

15. Verfahren nach Anspruch 14, wobei die Messvorrichtung (10) mit einer/der Außenmantelfläche (22.1) der Wandung (22) stoffschlüssig verbunden wird.

16. Verfahren nach Anspruch 14 oder 15, wobei der Drucksensor (32) an einer Radialposition zwischen Außenmantelfläche (22.1) und einer Innenmantelfläche (22.2) der Wandung angeordnet ist.

17. Verfahren nach einem der Ansprüche 14 bis 16, wobei der Drucksensor (32) in der Radialkavität (26) in einer Übermaßanpassung fluiddicht in der Wandung (22) positioniert ist.

18. Verfahren zur Messung einer Eigenschaft eines Fluids mittels einer medizintechnischen Messvorrichtung (10) nach einem der Ansprüche 1 bis 12, umfassend die Schritte:
- Führen eines Fluids, insbesondere Blut, durch eine Leitung (20) innerhalb einer von einer Wandung (22) umgrenzten Längskavität (24);
- Messen einer Eigenschaft, insbesondere des Drucks des Fluids mittels eines Drucksensors (32) der Messvorrichtung;
**dadurch gekennzeichnet, dass** die Eigenschaft in einer in die Wandung (22) eingebrachten Radialkavität (26) gemessen wird, in welcher der Drucksensor (32) und dessen Sensoroberfläche positioniert wird.

19. Verfahren zur Messung einer Eigenschaft eines Fluids nach Anspruch 18, wobei die Messung eine Druckmessung ist.

20. Verfahren zur Messung einer Eigenschaft eines Fluids nach Anspruch 18 oder 19, wobei der Drucksensor (32) in einer Radialposition zwischen einer Außenmantelfläche (22.1) und einer Innenmantelfläche (22.2) der Wandung (22) beabstandet von der Außenmantelfläche (22.1), bevorzugt in einer Radialposition näher zur Innenmantelfläche (22.2) als zur Außenmantelfläche (22.1) positioniert wird.

## Claims

1. Medico-technical measuring device (10) for measuring a property of a fluid, namely for pressure measurement, comprising
- a line (20) which extends along a central longitudinal axis (M) and is configured to guide a fluid, in particular blood, within a longitudinal cavity (24) delimited by a wall (22);
- a sensor unit (30) with a pressure sensor (32), which is configured to measure a property of the fluid guided in the longitudinal cavity (24);
wherein the sensor unit (30) has an abutting section (38), which abuts against the external sleeve surface (22.1) of the wall (22) and has a radial section (36),
wherein the line (20) is designed as a flexible hose line and has a radial cavity (26) introduced in the wall (22) in a radial direction, in which cavity the sensor unit (30) is at least partially arranged and is integrated in the wall (22), such that the pressure sensor (32) is in communication with the fluid,
**characterized in that**
the pressure sensor surface is positioned within the wall (22).

2. Measuring device (10) according to claim 1, wherein the measuring device (10) is configured to connect the sensor unit (30) in a single interface with the line (20), in particular to attach it directly to an external sleeve surface (22.1) of the wall (22).

3. Measuring device (10) according to one of the previous claims, wherein the sensor (32) is arranged in a radial position between an/the external sleeve surface (22.1) and an internal sleeve surface (22.2) of the wall inside the wall distanced from the external sleeve surface (22.1), in particular in a section which is arranged closer to the internal sleeve surface (22.2) than to the external sleeve surface (22.1).

4. Measuring device (10) according to one of the previous claims, wherein the radial cavity (26) is an opening, in particular a cylindrical bore with a uniform diameter.

5. Measuring device (10) according to one of the previous claims, wherein a diameter or an extent of a cross section of the radial cavity (26) is smaller than a section of the sensor unit (30) that corresponds geometrically thereto, wherein the radial cavity can, together with the sensor unit and/or the sensor (32), form an oversize fit to which the wall (22) can be sealed fluid-tight.

6. Measuring device (10) according to one of the previous claims, wherein the sensor unit (30) is configured to position the pressure sensor (32) in a predefined radial position in the radial cavity (26).

7. Measuring device (10) according to one of the previous claims, wherein the sensor unit (30) has an abutting section (38) which is designed to geometrically correspond to an/the external sleeve surface (22.1) of the wall (22), at least in the area of the radial cavity (26), in particular circumferentially around the radial cavity.

8. Measuring device (10) according to claim 7, wherein the sensor unit (30) is partially arranged in the radial cavity (26) in the wall (22) and integrated in the wall (22) such that the pressure sensor (32) is in communication with the fluid and such that the abutting section (38) of the sensor unit (30) is designed circumferentially around the radial cavity (26) and to geometrically correspond to an/the external sleeve surface (22.1) of the wall (22).

9. Measuring device (10) according to one of the previous claims, wherein an/the abutting section (38) of the sensor unit (30) in the area of the radial cavity (26), in particular circumferentially around the radial cavity, is connected by a substance-to-substance bond with an/the external sleeve surface (22.1) of the wall (22), in particular by means of an adhesive agent (12).

10. Measuring device (10) according to one of the previous claims, wherein the sensor unit (30) has a radial section (36) in which the pressure sensor (32) is arranged, wherein the radial section preferably has an extension in a radial direction, which is preferably larger than half of a wall thickness (r22) of the wall (22) in a section in which the radial cavity (26) is arranged.

11. Measuring device (10) according to one of the previous claims, wherein the sensor unit (30), at least partially, in particular at least a/the radial section (36) of the sensor unit, is made from a plastic material.

12. Measuring device (10) according to one of the previous claims **characterized in that** it is a disposable device provided for one-time use, wherein the sensor unit (30) preferably has a coupling point for a communication and/or energy supply, in particular for a transmission by wire over a cable or for a wireless transmission.

13. Measuring system (1) for extra-corporeal circulation with at least two measuring devices (10) according to one of the previous claims, wherein one of the measuring devices has a feed line (20), and another of the measuring devices has a discharge line (20).

14. Method for producing a medico-technical measuring device (10) according to one of the previous claims comprising the steps:
- providing a line (20) with a wall (22) as a flexible, pliable hose line;
- providing a sensor unit (30) with an abutting section (38) and a radial section (36), comprising a sensor mounting (34), in which the pressure sensor (32) is arranged;
- arranging a pressure sensor (32) at the line (20);
wherein a radial cavity (26) is inserted in the wall (22) in a radial direction, and the pressure sensor (32) is arranged in the radial cavity (26),
**characterized in that**
the sensor surface of the pressure sensor (32) is positioned in the radial cavity (26) and the sensor unit (30) with the abutting section (38) abuts against an external sleeve surface (22.1) of the wall (22).

15. Method according to claim 14, wherein the measuring device (10) is connected by a substance-to-substance bond with an/the external sleeve surface (22.1) of the wall (22).

16. Method according to claim 14 or 15, wherein the pressure sensor (32) is arranged at a radial position between the external sleeve surface (22.1) and the internal sleeve surface (22.2) of the wall.

17. Method according to one of claims 14 to 16, wherein the pressure sensor (32) in the radial cavity (26) is positioned by an oversize fit fluid-tight in the wall (22).

18. Method for measuring a property of a fluid by means of a medico-technical measuring device (10) according to one of claims 1 to 12, comprising the steps:
- guiding a fluid, in particular blood, through a line (20) within a longitudinal cavity (24) delimited by a wall (22);
- measuring a property, in particular the pressure of the fluid, by means of a pressure sensor (32) of the measuring device;
**characterized in that**
the property is measured in a radial cavity (26) inserted in the wall (22) in which the pressure sensor (32) and its sensor surface are positioned.

19. Method for measuring a property of a fluid according to claim 18, wherein the measurement is a pressure measurement.

20. Method for measuring a property of a fluid according to claim 18 or 19, wherein the pressure sensor (32) is positioned in a radial position between an external sleeve surface (22.1) and an internal sleeve surface (22.2) of the wall (22) distanced from the external sleeve surface (22.1), preferably in a radial position closer to the internal sleeve surface (22.2) than to the external sleeve surface (22.1).

## Revendications

1. Dispositif de mesure (10) lié à une technique médicale, destiné à la mesure d'une propriété d'un fluide, plus précisément à la mesure de la pression, comprenant :
- un conduit (20) qui s'étend le long d'un axe longitudinal médian (M) et qui est conçu pour guider un fluide, en particulier du sang, au sein d'une cavité longitudinale (24) délimitée par une paroi (22) ;
- un mécanisme de détection (30) comprenant un capteur de la pression (32), qui est conçu pour la mesure d'une propriété du fluide guidé dans la cavité longitudinale (24) ;
dans lequel le mécanisme de détection (30) présente un tronçon de placement (38) qui vient se placer contre la surface (22.1) de l'enveloppe extérieure de la paroi (22) et qui présente un tronçon radial (36) ;
dans lequel le conduit (20) est réalisé sous la forme d'un conduit souple flexible et présente une cavité radiale (26) incorporée en direction radiale dans la paroi (22), cavité dans laquelle est disposé au moins en partie le mécanisme de détection (30) et est intégré dans la paroi (22) d'une manière telle que le capteur de la pression (32) est mis en communication avec le fluide ; **caractérisé en ce que** la surface du capteur de la pression est disposée à l'intérieur de la paroi (22).

2. Dispositif de mesure (10) selon la revendication 1, dans lequel le dispositif de mesure (10) est conçu pour relier le mécanisme de détection (30) dans une interface unique au conduit (20), en particulier pour le fixer directement à une surface (22.1) de l'enveloppe externe de la paroi (22).

3. Dispositif de mesure (10) selon l'une quelconque des revendications précédentes, dans lequel le capteur (32) est disposé dans une position radiale, entre une/la surface (22.1) de l'enveloppe externe et une surface (22.2) du revêtement interne de la paroi à l'intérieur de la paroi, à distance de la surface (22.1) de l'enveloppe externe, en particulier dans un tronçon qui est disposé plus près de la surface (22.2) du revêtement interne que de la surface (22.1) de l'enveloppe externe.

4. Dispositif de mesure (10) selon l'une quelconque des revendications précédentes, dans lequel la cavité radiale (26) représente une ouverture, en particulier un alésage cylindrique possédant un diamètre homogène.

5. Dispositif de mesure selon l'une quelconque des revendications précédentes, dans lequel un diamètre ou une étendue d'une section transversale de la cavité radiale (26) est inférieur(e) à celui ou à celle d'un tronçon du mécanisme de détection (30) correspondant du point de vue géométrique ; dans lequel la cavité radiale forme conjointement avec le mécanisme de détection et/ou le capteur (32) un ajustement avec serrage contre lequel la paroi (22) peut être fermée de manière hermétique pour la rendre étanche aux fluides.

6. Dispositif de mesure (10) selon l'une quelconque des revendications précédentes, dans lequel le mécanisme de détection (30) est conçu pour positionner le capteur de la pression (32) dans une position radiale prédéfinie dans la cavité radiale (26).

7. Dispositif de mesure (10) selon l'une quelconque des revendications précédentes, dans lequel le mécanisme de détection (30) présente un tronçon de placement (38) qui est réalisé, au moins dans la zone de la cavité radiale (26), en particulier dans la zone qui entoure la cavité radiale, d'une manière correspondante du point de vue géométrique à une/la surface d'enveloppe externe (22.1) de la paroi (22).

8. Dispositif de mesure (10) selon la revendication 7, dans lequel le mécanisme de détection (30) est disposé en partie dans la cavité radiale (26) dans la paroi (22) et est intégré dans la paroi (22) d'une manière telle que le capteur de la pression (32) est mis en communication avec le fluide, et d'une manière telle que le tronçon de placement (38) du mécanisme de détection (30) est réalisé de façon à s'étendre à la périphérie de la cavité radiale (26) et de façon à correspondre du point de vue géométrique à une/la surface de l'enveloppe externe (22.1) de la paroi (22).

9. Dispositif de mesure (10) selon l'une quelconque des revendications précédentes, dans lequel un/le tronçon de placement (38) du mécanisme de détection (30) est relié, dans la zone de la cavité radiale (26), en particulier en s'étendant à la périphérie de la cavité radiale, par adhérence à une/la surface de l'enveloppe externe (22.1) de la paroi (22), en particulier au moyen d'un adhésif.

10. Dispositif de mesure (10) selon l'une quelconque des revendications précédentes, dans lequel le mécanisme de détection (30) présente un tronçon radial (36) dans lequel est disposé le capteur de la pression (32) ; dans lequel le tronçon radial présente en direction radiale de préférence une extension qui est de préférence supérieure à la moitié d'une épaisseur de paroi (r22) de la paroi (22) dans un tronçon dans lequel est disposée la cavité radiale (26).

11. Dispositif de mesure (10) selon l'une quelconque des revendications précédentes, dans lequel le mécanisme de détection (30) est réalisé au moins en partie, en particulier au moins un/le tronçon radial (36) du mécanisme de détection, à partir d'un matériau synthétique.

12. Dispositif de mesure (10) selon l'une quelconque des revendications précédentes, **caractérisé en ce qu'**il représente un dispositif jetable prévu pour l'usage unique ; dans lequel le mécanisme de détection (30) présente de préférence un endroit d'accouplement pour une communication et/ou une alimentation en énergie, en particulier pour une transmission par fil, par l'intermédiaire d'un câble, ou pour une transmission sans fil.

13. Système de mesure (1) pour la circulation extracorporelle, comprenant au moins deux dispositifs de mesure (10) selon l'une quelconque des revendications précédentes, dans lequel un des dispositifs de mesure présente un conduit d'alimentation (20) et un autre parmi les dispositifs de mesure représente un conduit d'évacuation (20).

14. Procédé pour la fabrication d'un dispositif de mesure (10) lié à une technique médicale, selon l'une quelconque des revendications précédentes, comprenant les étapes dans lesquelles :
- on procure un conduit (20) comprenant une paroi (22) sous la forme d'un conduit souple, flexible, apte à plier ;
- on procure un mécanisme de détection (30) comprenant un tronçon de placement (38) et un tronçon radial (36) comprenant un logement de capteur (34) dans lequel est disposé un capteur de la pression (32) ;
- on dispose un capteur de la pression (32) contre le conduit (20) ;
dans lequel on incorpore une cavité radiale (26) en direction radiale dans la paroi (22) et on dispose le capteur de la pression (32) dans la cavité radiale (26) ;
**caractérisé en ce que** la surface de détection du capteur de la pression (32) est positionnée dans la cavité radiale (26) et le mécanisme de détection (30) vient se placer avec un tronçon de placement (38) contre la surface de l'enveloppe externe (22.1) de la paroi (22).

15. Procédé selon la revendication 14, dans lequel le dispositif de mesure (10) est relié par adhérence à une/la surface de l'enveloppe externe (22.1) de la paroi (22).

16. Procédé selon la revendication 14 ou 15, dans lequel le capteur de la pression (32) est disposé à une position radiale entre la surface de l'enveloppe externe (22.1) et une surface du revêtement interne (22.2) de la paroi.

17. Procédé selon l'une quelconque des revendications 14 à 16, dans lequel le capteur de la pression (32) est disposé dans la cavité radiale (26) dans un ajustement avec serrage d'une manière étanche aux fluides, dans la paroi (22).

18. Procédé pour la mesure d'une propriété d'un fluide au moyen d'un dispositif de mesure (10) lié à une technique médicale, selon l'une quelconque des revendications 1 à 12, comprenant les étapes dans lesquelles :
- on guide un fluide, en particulier du sang, au sein d'une cavité longitudinale (24) délimitée par une paroi (22) ;
- on mesure une propriété, en particulier la pression du fluide au moyen d'un capteur de la pression (32) du dispositif de mesure ;
**caractérisé en ce qu'**on mesure la propriété dans une cavité radiale (26) incorporée dans la paroi (22), cavité dans laquelle sont disposés le capteur de la pression (32) et la surface de détection de ce dernier.

19. Procédé pour la mesure d'une propriété d'un fluide selon la revendication 18, dans lequel la mesure est une mesure de la pression.

20. Procédé pour la mesure d'une propriété d'un fluide selon la revendication 18 ou 19, dans lequel le capteur de la pression (32) est disposé dans une position radiale entre une surface de l'enveloppe externe (22.1) et une surface du revêtement interne (22.2) de la paroi (22) à l'écart de la surface de l'enveloppe externe (22.1), de préférence dans une position radiale plus proche de la surface du revêtement interne (22.2) que de la surface de l'enveloppe externe (22.1).
